# EUROPÄISCHE PATENTSCHRIFT

(11) **EP 2 425 822 B1**
(45) Veröffentlichungstag und Bekanntmachung des Hinweises auf die Patenterteilung: **19.12.2012**
(21) Anmeldenummer: 11191245.7
(22) Anmeldetag: 24.10.2008
(51) Int. Cl.: A61K 9/16, A61K 9/20, A61K 31/485

(54) **Retardtabletten mit Hydromorphon**
Sustained release tablets with hydromorphone
Comprimés à libération prolongée contenant de l'hydromorphone

(30) Priorität: 09.11.2007 EP 07021788
(43) Veröffentlichungstag der Anmeldung: 07.03.2012
(62) Teilanmeldung aus: 08847385.5
(73) Patentinhaber: Acino Pharma AG, 4253 Liesberg (CH)
(72) Erfinder: Reher, Markus, 4102 Binningen (CH); Zingraff, Marc, 68300 Saint-Louis (FR)
(74) Vertreter: Best, Michael

(56) Entgegenhaltungen:
- EP-A- 0 271 193
- WO-A-2004/112746
- US-A1- 2001 038 856
- US-A1- 2005 074 493
- US-A1- 2006 039 976
- US-A1- 2007 104 789
- US-A1- 2007 207 200

## Beschreibung

Die vorliegende Erfindung betrifft Tabletten mit einem Tablettenkern aus mehreren wirkstoffhaltigen Pellets, sogenannte "MUPS"-Tabletten sowie Pellets, die insbesondere zur Herstellung derartiger MUPS-Tabletten geeignet sind. Die erfindungsgemäßen Arzneimittel enthalten als Wirkstoff Hydromorphon und zeichnen sich insbesondere dadurch aus, dass die Pellets Retardpellets sind, bei denen die Retardierung über eine auf der Wirkstoffschicht aufgebrachte Schicht erfolgt, über dieser Retardschicht aber noch eine schnell freisetzende wirkstoffhaltige Beschichtung aufgebracht ist.

Arzneimittel mit dem Wirkstoff Hydromorphon sind schon seit langem bekannt. Es ist ebenfalls bekannt, Hydromorphon über Retardformulierungen, bei denen der Wirkstoff langsam über einen längeren Zeitraum hinweg mit einem bestimmten Freisetzungsprofil freigesetzt wird, zu verabreichen. Derartige Arzneimittel sind beispielsweise in der EP-A 271 193 beschrieben. Diese Druckschrift offenbart beispielhaft Tabletten, bei denen Hydromorphonhydrochlorid in einer retardierenden Matrix formuliert wird. Die Druckschrift offenbart in allgemeiner Form ebenfalls, dass das Arzneimittel als Sphäroid vorliegen kann, das mit einer die Freisetzung kontrollierenden Filmbeschichtung versehen ist. Die Filmbeschichtung wird so gewählt, dass ein bestimmtes *in vitro*-Freisetzungsprofil erzielt wird. Eine Offenbarung dahingehend, dass die Pellets mit üblichen Hilfsstoffen zu MUPS-Tabletten verpresst werden, findet sich in dieser Druckschrift nicht.

Die EP-A 548 448 offenbart, dass es bei Retardformulierungen, bei denen der Wirkstoff als Beschichtung auf einem inerten Kern vorliegt, häufig zu Stabilitätsproblemen kommen kann, wenn die Beschichtung aus einen wässrigen System heraus aufgetragen wird. Als Beispiele für derartige Retardformulierungen werden auch Formulierungen mit dem Wirkstoff Hydromorphon angegeben, insbesondere betreffen die meisten Beispiele der Druckschrift Hydromorphon. Zur Lösung dieser Stabilitätsprobleme schlägt die Druckschrift vor, die Pellets einer besonderen Härtungsreaktion zu unterziehen. Probleme, die bei der Herstellung von MUPS-Tabletten auftreten können, werden in der EP-A 548 448 nicht beschrieben.

Es ist bekannt, bei Retardformulierungen einen schnell freisetzenden Bestandteil zur Verfügung zu stellen, um ein schnelles Anfluten des Wirkstoffes zu erzielen. Liegt die Retardformulierung in Form von Pellets vor, wird dies beispielsweise dadurch bewirkt, dass zusammen mit den retardierenden Pellets den Wirkstoff schnell freisetzende Pellets formuliert werden. Es ist auch bekannt, beispielsweise über eine Retardmatrix, die den Wirkstoff enthält, eine schnell freisetzende Beschichtung mit dem Wirkstoff vorzusehen, um ein schnelles Anfluten des Wirkstoffes zu gewährleisten. Diesbezüglich kann beispielsweise auf Remington: The Science and Practice of Pharmacy, 2000, Seite 904 und Robinson, Drugs and the pharmaceutical Sciences, Volume 6: Sustained and Controlled Release Drug Delivery Systems, 1978, Seite 139, verwiesen werden.

Im Falle des Wirkstoffes Hydromorphon sind bislang noch keine Formulierungen bekannt, bei denen ein schnell freisetzender Anteil neben einer retardierenden Formulierung vorgesehen ist. Offensichtlich ist zur Erzielung des im Stand der Technik gewünschten Freisetzungsprofils, wie es zum Beispiel in der EP-A 271 193 beschrieben wird, ein derartiger schnell freisetzender Bestandteil nicht erforderlich und/oder nicht erwünscht.

Versucht man Pellets mit dem Wirkstoff Hydromorphon, bei denen eine Wirkstoffschicht auf einen inerten Kern aufgebracht ist und die Retardierung über eine Retardschicht über der Wirkstoffschicht bewirkt wird, zu MUPS-Tabletten zu formulieren, also zusammen mit üblichen Hilfs- und Zusatzstoffen zu Tabletten zu verpressen, treten Probleme auf. Wie in vielen Schutzrechten des Standes der Technik beschrieben, besteht bei diesen Verfahren die Gefahr, dass die funktionelle Beschichtung der Pellets, d.h. im vorliegenden Fall die Retardbeschichtung beschädigt wird, was zu einer nicht kontrollierten und nicht reproduzierbaren Veränderung des Freisetzungsprofils führt und damit erhebliche Risiken für den Patienten mit sich bringt.

Der vorliegenden Erfindung liegt die Aufgabe zugrunde, Pellets und daraus hergestellte MUPS-Tabletten zur Verfügung zu stellen, die die genannten Probleme nicht aufweisen und darüber hinaus ein vorteilhaftes Freisetzungsverhalten und eine gute Stabilität zur Verfügung stellen.

Diese Aufgabe wird durch den Gegenstand der Patentansprüche gelöst.

Die erfindungsgemäßen Pellets und damit auch die erfindungsgemäßen Tabletten enthalten als Wirkstoff Hydromorphon. Bevorzugt ist Hydromorphon der einzige Wirkstoff, der in den erfindungsgemäßen Pellets und den erfindungsgemäßen Tabletten vorhanden ist. Der Wirkstoff ist in den Tabletten bevorzugt in einer Konzentration im Bereich von 0.5 bis 25 Gew.-%, insbesondere im Bereich von 0.5 Gew.-% bis 15 Gew.-%, bezogen auf das Gesamtgewicht der Tablette enthalten. Bevorzugt enthält eine erfindungsgemäße Tablette Hydromorphon im Bereich von 1 bis 100 mg, insbesondere im Bereich von 2 bis 50 mg, stärker bevorzugt im Bereich von 2 bis 40 mg, beispielsweise im Bereich von 4 mg bis 30 mg, am stärksten bevorzugt im Bereich von 4 mg bis 24 mg.

Der Wirkstoff liegt bevorzugt als Hydrochlorid vor, er kann aber auch als freie Base, als ein anderes Salz oder als Solvat oder als Solvat eines Salzes vorliegen. Wenn im Rahmen dieser Anmeldung von dem Wirkstoffgehalt gesprochen wird, bezieht sich dieser immer auf das Gewicht des Salzes oder Solvats, falls ein Salz oder Solvat eingesetzt wird. Unter einem Solvat des Wirkstoffes wird auch ein Solvat des Salzes des Wirkstoffes verstanden.

Die erfindungsgemäßen Pellets weisen einen inerten Kern auf. Derartige inerte Kerne sind im Stand der Technik bekannt und werden beispielsweise als Non-Pareil in verschiedenen Größen vertrieben. Als Beispiel kann hier das Produkt Non-Pareil 18-20 (mesh) benannt werden. Allgemein weisen derartige inerte Kerne einen Durchmesser im Bereich von 0,2 mm bis 2,5 mm, insbesondere im Bereich von 0,2 mm bis 1,5 mm auf. Sie sind auch unter der Bezeichnung "Neutralkerne" bekannt. Häufig werden Zuckerkerne oder Kerne aus mikrokristalliner Cellulose als Neutralkerne verwendet, aber auch andere Neutralkerne sind in der Fachwelt bekannt.

Erfindungsgemäß befindet sich auf den inerten Kernen eine Wirkstoffschicht, bei der der Wirkstoff, d.h. das Hydromorphon mit einem oder mehreren Bindemitteln als Beschichtung auf den inerten Kern aufgebracht ist. Diese Beschichtung ist bevorzugt nicht retardierend, d.h. aus ihr wird das Hydromorphon schnell freigesetzt, d.h. mindestens 90% innerhalb von 15 Minuten, bestimmt nach der Paddle-Methode der U.S.-Pharmacopoea (100 rpm in 900 ml wässrigem Puffer, pH im Bereich von 1,6 und 7,2 bei 37°C). Sofern nichts anderes angegeben ist, beziehen sich alle in dieser Anmeldung genannten Freisetzungsdaten auf *in vitro*-Freisetzungen, die nach diesem Verfahren der U.S.-Pharmacopoea erhalten werden.

Diese Wirkstoffschicht, die sich auf dem inerten Kern befindet, wird im Rahmen dieser Anmeldung als "innere" Wirkstoffschicht bezeichnet. Die innere Wirkstoffschicht enthält in der Regel ein Bindemittel und den Wirkstoff und kann neben dem Bindemittel und dem Wirkstoff auch noch weitere übliche pharmazeutisch verträgliche Hilfs- und Zusatzstoffe enthalten. Derartige Stoffe sind dem Fachmann bekannt. Geeignete Bindemittel sind beispielsweise niedrigviskose wasserlösliche Polymere, insbesondere wasserlösliche Hydroxyniedrigalkylcellulosen wie Hydroxypropylcellulose, niedrig substituierte Hydroxypropylcellulose, Hydroxypropylmethylcellulose etc. Weitere geeignete Bindemittel sind Aminoalkyl-Methacrylat Copolymere, Gelatine, Gummi Arabicum, Guar gum, Methylcellulose, Carboxymethylcellulose, Ethylhydroxyethylcellulose, Hydroxyethylmethylcellulose, Hydroxyethylcellulose, Gum Tragant, Polyvinylpyrrolidon, Polyvinylacetat, Polyvinylalkohol sowie anorganische Gele, aber auch Dextrin, Natriumalginat, Pektin etc.

Die innere Wirkstoffschicht kann ebenfalls beispielsweise Farbstoffe, Weichmacher wie Triethylcitrat, Polyethylenglykol oder weitere Hilfsstoffe enthalten.

Auf der inneren Wirkstoffschicht befindet sich die Schicht, die die Freisetzung steuert. Derartige Schichten die die Freisetzung des Wirkstoffs steuern sind im Stand der Technik bekannt und es kann beispielsweise wiederum auf die EP-A 271 193 oder auf die EP-A 553 392 verwiesen werden. In der Regel weist diese Schicht ein Gemisch aus einem wasserunlöslichen Polymer und einem wasserlöslichen Polymer auf. Als wasserlösliches Polymer kommen im Prinzip all diejenigen wasserlöslichen Polymere in Betracht, die vorstehend als Bindemittel für die innere Wirkstoffschicht genannt werden. Besonders bevorzugt wird als wasserlösliches Material z.B. Hydroxypropylmethylcellulose oder eine andere wasserlösliche Cellulose oder Polyvinylpyrrolidon oder ein ähnliches Material verwendet. Als wasserunlösliches Polymer wird Ethylcellulose verwendet. Dieses Material ist bekannt und neben den vorstehend genannten Druckschriften beispielsweise auch in der EP-A 722 730 beschrieben. Auch die Abmischung des wasserunlöslichen Polymers mit dem wasserlöslichen Polymer erfolgt wie im Stand der Technik bekannt. Die auf der Wirkstoffschicht aufgebrachte Retardbeschichtung kann wie die innere Wirkstoffschicht weitere übliche pharmazeutisch verträgliche Hilfs- und Zusatzstoffe wie Farbstoffe, Weichmacher wie Triethylcitrat etc. enthalten.

Sofern die Pellets nicht zu MUPS-Tabletten verpresst werden sollen sondern beispielsweise in Kapseln abgefüllt werden, sind die vorstehend beschriebenen Pellets mit einem inerten Kern, einer inneren Wirkstoffschicht und einer retardierenden Beschichtung bereits verwendbar, es ist nicht erforderlich, noch eine weitere Beschichtung vorzusehen. Mit diesen Pellets kann bereits ein vorteilhaftes Freisetzungsprofil und eine vorteilhafte Freisetzung des Hydromorphons erreicht werden, wenn sie in Kapseln, Sachets etc. abgefüllt und verabreicht werden.

Sollen die Pellets zu MUPS-Formulierungen verpresst werden, hat es sich erfindungsgemäß jedoch überraschend als vorteilhaft herausgestellt, über der retardierenden Beschichtung noch eine weitere Wirkstoffschicht vorzusehen, die im Rahmen dieser Anmeldung als "äußere" Wirkstoffschicht bezeichnet wird. Die Zusammensetzung der äußeren Wirkstoffschicht ist im Prinzip wie die Zusammensetzung der inneren Wirkstoffschicht und bevorzugt weist sowohl die äußere als auch die innere Wirkstoffschicht die gleichen Bestandteile auf.

Dadurch, dass die Pellets über der retardierenden Schicht auch eine schnell freisetzende wirkstoffhaltige Beschichtung aufwiesen, können sie leicht zu MUPS-Tabletten verpresst werden, die Gefahr, dass bei dem Verpressen eine Beschädigung der Beschichtungen dergestalt auftritt, dass sich hierdurch das Freisetzungsprofil der Pellets unkontrolliert ändert, wird deutlich verringert. Dieser Effekt wurde im Stand der Technik für eine schnell freisetzende Wirkstoffbeschichtung auf einer Retardbeschichtung noch nicht beschrieben und ist überraschend.

Der Gehalt an Hydromorphon in der inneren Wirkstoffschicht beträgt bevorzugt 2 mg bis 80 mg, stärker bevorzugt 2,4 mg bis 45 mg, insbesondere 2,8 mg bis 23,8 mg. Bevorzugt befindet sich 50% bis 99% des gesamten Wirkstoffgehaltes in der inneren Wirkstoffschicht, stärker bevorzugt befindet sich 60% bis 99% des gesamten Wirkstoffgehaltes in der inneren Wirkstoffschicht und insbesondere befindet sich 70% bis 99% des gesamten Wirkstoffes in der inneren Wirkstoffschicht.

In der äußeren Wirkstoffschicht befindet sich bevorzugt 0,04 mg bis 12 mg Wirkstoff, stärker bevorzugt 0,04 mg bis 9,6 mg und insbesondere 0,04 mg bis 7,2 mg. Die bevorzugten prozentualen Anteile des Wirkstoffes in der äußeren Wirkstoffschicht ergeben sich durch Subtraktion der vorstehend angegebenen prozentualen Anteile des Wirkstoffs in der inneren Wirkstoffschicht von 100%.

Die innere Wirkstoffschicht hat bevorzugt eine Dicke im Bereich von 10 µm bis 200 µm, stärker bevorzugt im Bereich von 10 µm bis 100 µm.

Die Retardierungsschicht hat bevorzugt eine Dicke im Bereich von 10 µm bis 200 µm, stärker bevorzugt im Bereich von 10 µm bis 100 µm.

Die äußere Wirkstoffschicht hat bevorzugt eine Dicke im Bereich von 10 µm bis 100 µm, stärker bevorzugt im Bereich von 10 µm bis 80 µm.

Die Gesamtpellets haben vorzugsweise einen Durchmesser im Bereich von 200 µm bis 3000 µm, stärker bevorzugt im Bereich von 200 µm bis 2000 µm.

Erfindungsgemäß ist es möglich, dass die Pellets neben den beschriebenen Schichten noch weitere Schichten aufweisen. Beispielsweise können der inerte Kern und die innere Wirkstoffschicht oder auch die innere Wirkstoffschicht und die Retardschicht, aber auch die Retardschicht und die äußere Wirkstoffschicht noch jeweils durch eine Zwischenschicht getrennt sein. Ferner können auf der äußeren Wirkstoffschicht noch weitere Beschichtungen vorhanden sein. Die Zusammensetzung derartiger Zwischenschichten bzw. äußeren Beschichtungen ist dem Fachmann bekannt, sie bestehen beispielsweise aus einem Bindemittel wie insbesondere einem wasserlöslichen Cellulosepolymer und gegebenenfalls üblichen pharmazeutisch unbedenklichen Hilfs- und Zusatzstoffen. Wesentlich ist, dass diese zusätzlichen Schichten die Freisetzungseigenschaften der Pellets nicht negativ beeinflusst. Erfindungsgemäß bevorzugt enthalten die wirkstoffhaltigen Pellets jedoch keine weiteren Schichten und bestehen aus dem inerten Kern, der inneren Wirkstoffschicht, der Retardschicht und der äußeren Wirkstoffschicht.

Die Retardpellets mit einer inneren und einer äußeren Wirkstoffschicht können mit üblichen pharmazeutisch verträglichen Hilfsstoffen zu einem Tablettenkern verpresst werden. Die Hilfsstoffe zur Herstellung derartiger MUPS-Tabletten sind dem Fachmann bekannt, diesbezüglich kann z.B. auf das Standardwerk von Ritschel und Bauer-Brandl, "Die Tablette", Edition Cantor Verlag, 2002, verwiesen werden, das durch Bezugnahme aufgenommen wird. In der Regel werden zur Herstellung der Tabletten Füllstoffe, Bindemittel und Sprengmittel verwendet, gegebenenfalls auch Schmiermittel, Gleitmittel und Gemische davon. Selbstverständlich können auch Geschmacksstoffe, Färbemittel und weitere Hilfsmittel vorhanden sein. Bevorzugt enthalten die erfindungsgemäßen Tabletten neben den wirkstoffhaltigen Pellets noch mindestens einen Füllstoff, stärker bevorzugt mindestens einen Füllstoff und mindestens ein Sprengmittel, noch stärker bevorzugt mindestens einen Füllstoff, mindestens ein Sprengmittel und mindestens ein Bindemittel. Bevorzugt sind ebenfalls noch Schmiermittel und Gleitmittel vorhanden.

Als Bindemittel können die gleichen Bindemittel genannt werden wie sie vorstehend im Zusammenhang mit der inneren wirkstoffhaltigen Schicht offenbart wurden.

Geeignete Füllstoffe sind beispielsweise Laktose, wobei modifizierte Laktose oder wasserfreie (NF) Laktose genannt werden kann, Stärke, insbesondere modifizierte (pregelatinisierte) Stärke, native Stärke oder Gemische von beidem, Calciumphosphat, insbesondere dibasisches, ungemahlenes dibasisches und wasserfreies dibasisches Calciumphosphat, Cellulosederivate, Cellulose, insbesondere mikrokristalline Cellulose, Mannit, Sorbit, etc.

Selbstverständlich können Gemische verschiedener Füllstoffe verwendet werden.

Geeignete Sprengmittel sind beispielsweise Polyvinylpolypyrrolidon (PVPP), Agar, Kartoffelstärke, Formaldehyd-Casein, Natriumcarboxymethylamylopektin, Bentonit, Natriumalginat, Natriumcarboxymethylcellulose, hochdisperses Siliziumdioxid oder auch Trockenpektin. Wie bei den Bindemitteln und bei den Füllstoffen können selbstverständlich auch bei den Sprengmitteln Gemische verschiedener Sprengmittel verwendet werden.

Geeignete Fließreguliermittel sind erfindungsgemäß bekannt, hierbei kann es sich beispielsweise um "Gleitol", Talk, kolloidales Siliziumdioxid, gefälltes Silica, Calciumstearat, Magnesiumstearat, Stearinsäure, Laurylsäure, Stearylalkohol, Palmitinsäure, Beheninsäure, Caprinsäure, Carbowax oder Aerosil handeln.

Auch geeignete Schmiermittel sind dem Fachmann bekannt, wobei viele Verbindungen, die als Fließregulierungsmittel geeignet sind, auch als Schmiermittel verwendet werden können. Geeignete Schmiermittel sind beispielsweise Calciumstearat, Beheninsäure, Stearinsäure, Aluminiumstearat, Stearylalkohol, hydriertes Rizinusöl, Palmitinsäure, Cetylalkohol, Talk, Magnesiumstearat, Myoistinsäure, Lanette O, Laurylsäure, entfettetes Milchpulver, Gleitol, Talkumin, Caprinsäure, Bolus Alba, Stärke und Polyethylenglykole, wie Carbowax 6000.

Erfindungsgemäß bevorzugt weisen die Kerne der MUPS-Tabletten mindestens 10%, stärker bevorzugt mindestens 20%, noch stärker bevorzugt mindestens 30%, insbesondere mindestens 40%, beispielsweise mindestens 50% an üblichen Hilfsstoffen auf, wobei der Rest die wirkstoffhaltigen Pellets ausmacht. Die wirkstoffhaltigen Pellets machen erfindungsgemäß bevorzugt aber mindestens 20%, stärker bevorzugt mindestens 30% der Tablettenkerne der MUPS-Tabletten aus.

Der folgenden Tabelle können bevorzugte Hilfsstoffe und deren bevorzugte Menge in der MUPS-Tablette entnommen werden, sofern der jeweilige Hilfsstoff in der Tablette eingesetzt wird (Rest Wirkstoffpellets).

| **Hilfsstoffe** | **bevorzugt** | **besonders bevorzugt** | **am stärksten bevorzugt** |
|---|---|---|---|
| Füllstoffe (20 bis 90%, bezogen auf das Gewicht der Filmtablette) | Laktose, Cellulose, Stärke, Phosphatsalze, Mannit, Maltose, Maltodextrin, Sorbit, Sucrose | Laktose, Cellulose, Stärke, Phosphatsalze | Cellulose, Laktose |
| Bindemittel (0,5 bis 25%, bezogen auf das Gewicht der Filmtablette) | Dextrin, Dextrate, Dextrose, Cellulosederivate, Gelatine, Gums, Polyvinylpyrrolidon, Stärke, Sucrose | Cellulosederivate, Polyvinylpyrrolidon, Stärke | Polyvinylpyrrolidon, Cellulosederivate |
| Sprengmittel (1 bis 25%, bezogen auf das Gewicht der Filmtablette) | PVPP, Agar, Bentonit, Carboxymethylcellulose, Natriumalginate, Stärke | PVPP, Carboxymethylcellulose | PVPP, Carboxymethyl-cellulose |
| Gleitmittel (0,2 bis 10%, bezogen auf das Gewicht der Filmtablette) | Magnesiumstearat, hydriertes Rizinusöl, Glycerylester, Polyethylenglykol, Natriumstearylfumarat, Stearinsäure, Talkum | Magnesiumstearat, hydriertes Rizinusöl, Natriumstearylfumarat | Magnesiumstearat, Rizinusöl |
| Fließreguliermittel (0,1 bis 15%, bezogen auf das Gewicht der Filmtablette) | Kolloidales Siliziumdioxid, gefälltes .. Silica, Stärke, Talkum, Stearinsäure, Palmitinsäure, gepulverte Cellulose | Kolloidales Siliziumdioxid, gefälltes Silica | Kolloidales Siliziumdioxid |
| Farbstoffe (0,01 bis 5%, bezogen auf das Gewicht der Filmtablette) | FD&C und D&C blue, green, orange, red, violet, yellow, E 100 bis 180 | FD&C und D&C blue, green, Titandioxid E 171, E 127 erythrosine, E 131 Patent blau | Titandioxid E 171 |
| sonstige Hilfsstoffe (0,1 bis 10%, bezogen auf das Gewicht der Filmtablette) | Triethylcitrat, Dibutylsebacat, Propylenglykol, Diethylphthalat, Dibutylphthalat, Glycerinmonostearat, Triacetin, Stearinsäure | Triethylcitrat, Dibutylsebacat, Glycerinmonostearat, Stearinsäure | Propylenglykol, Triethylcitrate, Dibutylsebacat |

Die Tablettenkerne der MUPS-Tabletten sind mit einer üblichen Beschichtung versehen, wie es im Stand der Technik bekannt ist. Diese Beschichtung soll keinen Einfluss auf die Freisetzung des Hydromorphons haben, sie ist daher in der Regel wasserlöslich und basiert auf einem wasserlöslichen Bindemittel wie es vorstehend im Zusammenhang mit der inneren Wirkstoffschicht beschrieben wurde und üblichen Hilfs- und Zusatzstoffen. Als Bindemittel sind wiederum wasserlösliche Celluloseether wie HPC, HPMC etc. sowie z.B. PVP bevorzugt. Das Aufbringen derartiger Beschichtungen ist dem Fachmann bekannt, es kann wiederum auf das bereits erwähnte Standardwerk "Die Tablette" verwiesen werden.

Die Pellets werden bevorzugt zu MUPS-Tabletten verpresst, es ist aber selbstverständlich auch möglich, die Pellets zu Kapseln, Sachets oder anderen geeigneten Verabreichungsformen zu verarbeiten, wie es im Stand der Technik bekannt ist.

Die Herstellung der erfindungsgemäßen Pellets erfolgt nach im Stand der Technik üblichen Methoden.

Die Herstellung der Pellets erfolgt in 3 Schritten:

### 1. Wirkstoffbeladung der Kerne

Die verschiedenen Hilfsstoffe und der Wirkstoff werden im Lösungs-/Suspendiermittel gelöst/suspendiert. Die Lösung/Suspension wird auf die Kerne in einem Wirbelschichtgerät gesprüht.

### 2. Retardierung der Wirkstoffpellets

Die verschiedenen Hilfsstoffe werden im Lösungs-/Suspendiermittel gelöst/suspendiert. Die Lösung/Suspension wird auf die Wirkstoffpellets in einem Wirbelschichtgerät gesprüht.

### 3. Überzug der retardierenden Wirkstoffpellets mit einer zusätzlichen Wirkstoffschicht

Die verschiedenen Hilfsstoffe und der restliche Wirkstoff werden im Lösungs-/Suspendiermittel gelöst/suspendiert. Die Lösung/Suspension wird auf die Retardpellets in einem Wirbelschichtgerät aufgebracht.

Auch das Verpressen der erfindungsgemäßen Pellets zu MUPS-Tabletten erfolgt auf im Stand der Technik bekannte Art und Weise, beispielsweise wie folgt.

Die fertigen Pellets werden mit anderen Hilfsstoffen in einen geeigneten Mischer gemischt, bis die Mischung homogen ist. Die Mischzeiten, aber auch die Korngrößenverteilung der verschiedenen Hilfsstoffe, insbesondere der Füllmittel, werden von einem Fachmann geeignet eingestellt.

Die sogenannte Endmischung wird danach auf einer Tablettepresse tablettiert. Die Tablettiergeschwindigkeit und Tablettierdruck der Tablettenkerne werden von einem Fachmann geeignet eingestellt.

Die Tablettenkerne werden dann mit einem nicht-funktionalen Lack überzogen. Die verschiedenen Hilfsstoffe werden im Lösungs-/Suspendiermittel gelöst/suspendiert. Die Lösung/Suspension wird auf den Tablettenkernen in einem geeigneten Gerät besprüht (entweder Air-Coater oder Trommel-Coater).

Das Freisetzungsprofil von Hydromorphon aus den erfindungsgemäßen Pellets bzw. MUPS-Tabletten ist so wie es im Stand der Technik für die bekannten Hydromorphon-Formulierungen beschrieben wird und diesbezüglich kann insbesondere auf die EP-A 548 448 und die EP-A 271 193 verwiesen werden, deren Offenbarungsgehalt insoweit durch Bezugnahme aufgenommen wird.

Die folgenden Beispiele erläutern die Erfindung.

### Beispiel zur Herstellung von Pellets:

1. Polyethylenglykol wird mit Hydroxypropylmethylcellulose und Hydromorphon HCl in Wasser gelöst. Talkum wird separat in Wasser suspendiert, und danach zur Hydromorphonlösung gegeben. Die resultierende Suspension wird auf Zuckerpellets bei einer Produkttemperatur von 39 - 45°C in einen Glatt Wirbelschichtgerät aufgesprüht.
2. Ethylcellulose wird zusammen mit Propylenglykol, und Hydroxypropylcellulose in Ethanol gelöst. Zusätzlich kann man Talkum separat in Wasser oder Ethanol suspendieren, und zur Ethylcelluloselösung zugeben. Die resultierende Lösung/Suspension wird auf die Hydromorphon HCl Wirkstoffpellets bei einer Produkttemperatur von 39 - 50°C im Glatt Wirbelschichtgerät aufgesprüht.
3. Polyethylenglykol wird mit Hydroxypropylmethylcellulose und Hydromorphon HCl in Wasser gelöst. Talkum wird separat in Wasser suspendiert, und wird danach zur Hydromorphonlösung gegeben. Die resultierende Suspension wird auf Retardpellets bei einer Produkttemperatur von 39 - 45°C im Glatt Wirbelschichtgerät aufgesprüht.

### Beispiel zur Herstellung von MUPS Tabletten:

1. Die fertigen Pellets werden mit mikrokristalliner Cellulose und gesiebtem kolloidalem Siliziumdioxid gemischt. Gesiebtes Magnesiumstearat wird anschließend zugegeben und die Mischung wird weiter gemischt.
2. Die Endmischung wird tablettiert. Die Tablettiergeschwindigkeit wird so eingestellt, dass die Endmischung bei der Tablettierung homogen bleibt. Der Tablettierungsdruck wird geeignet eingestellt.
3. Die Lackiersuspension wird wie folgend hergestellt. Hydroxypropylmethylcellulose wird mit Polyethylenglykol in Wasser gelöst. Talkum wird separat mit Titandioxid in Wasser suspendiert, und danach zur Hydroxypropylmethylcelluloselösung gegeben. Die resultierte Suspension wird auf den Tablettenkernen bei einer Produkttemperatur von 39 - 45°C in einen Glatt Coater gesprüht.

## Patentansprüche

1. Verfahren zur Herstellung einer Tablette mit einem Tablettenkern aus mehreren wirkstoffhaltigen Pellets und einem oder mehreren pharmazeutisch verträglichen Hilfsstoffen und mindestens einer auf dem Tablettenkern aufgebrachten Beschichtung, umfassend das Aufbringen einer weiteren Wirkstoffschicht auf wirkstoffhaltigen Pellets die folgenden Aufbau aufweisen:
a) ein inerter Kern,
b) eine auf dem inerten Kern aufgebrachte Wirkstoffschicht und
c) eine auf der Wirkstoffschicht aufgebrachte Schicht, die die Freisetzung des Wirkstoffs retardiert,
wobei der Wirkstoff Hydromorphon ist und die auf der Wirkstoffschicht aufgebrachte Schicht, die die Freisetzung des Wirkstoffs retardiert, als Retardierungsmittel Ethylcellulose enthält.

2. Tablette mit einem Tablettenkern aus mehreren wirkstoffhaltigen Pellets und einem oder mehreren pharmazeutisch verträglichen Hilfsstoffen und mindestens einer auf dem Tablettenkern aufgebrachten Beschichtung, wobei die wirkstoffhaltigen Pellets als Wirkstoff Hydromorphon oder ein Salz oder ein Solvat davon enthalten und folgenden Aufbau aufweisen:
a) ein inerter Kern,
b) eine auf dem inerten Kern aufgebrachte Wirkstoffschicht,
c) eine auf der Wirkstoffschicht aufgebrachte Schicht, die die Freisetzung des Wirkstoffs retardiert und
d) auf der die Freisetzung des Wirkstoffs retardierenden Schicht eine weitere Wirkstoffschicht,
wobei die auf der Wirkstoffschicht aufgebrachte Schicht, die die Freisetzung des Wirkstoffs retardiert, als Retardierungsmittel Ethylcellulose enthält.

3. Tablette nach Anspruch 2, die den Wirkstoff in einer Konzentration im Bereich von 2 bis 30 Gew.-% enthält, bezogen auf das Gesamtgewicht der Tablette.

4. Tablette nach Anspruch 2 oder 3, bei der die pharmazeutisch verträglichen Hilfsstoffe, die mit den Pellets zu dem Tablettenkern verpresst sind, ausgewählt sind aus Bindemitteln, Füllstoffen, Sprengmitteln, Schmiermitteln, Gleitmitteln und Gemischen davon.

5. Tablette nach einem der vorstehenden Ansprüche, bei der eine Beschichtung auf dem Tablettenkern aufgebracht ist und diese Beschichtung keine die Freisetzung des Wirkstoffs retardierende Schicht ist.

6. Wirkstoffhaltiges Pellet mit dem Wirkstoff Hydromorphon, das folgenden Aufbau aufweist:
a) ein inerter Kern,
b) eine auf dem inerten Kern aufgebrachte Wirkstoffschicht und
c) eine auf der Wirkstoffschicht aufgebrachte Schicht, die die Freisetzung des Wirkstoffs retardiert,
wobei die auf der Wirkstoffschicht aufgebrachte Schicht, die die Freisetzung des Wirkstoffs retardiert, als Retardierungsmittel Ethylcellulose enthält.

7. Wirkstoffhaltiges Pellet nach Anspruch 6, **dadurch gekennzeichnet, dass** der inerte Kern einen Durchmesser im Bereich von 0,2 mm bis 1,5 mm aufweist.

8. Wirkstoffhaltiges Pellet nach Anspruch 6 oder 7, **dadurch gekennzeichnet, dass** die Wirkstoffschicht mindestens 90 % des Wirkstoffs innerhalb von 15 Minuten freisetzt, bestimmt nach der Paddle-Methode der US Pharmacopeia (100 rpm in 900 ml wässrigem Puffer, pH im Bereich von 1,6 und 7,2 bei 37°C).

## Claims

1. Method for the production of a tablet having a tablet core consisting of several active ingredient-containing pellets and one or more pharmaceutically acceptable excipients and at least one coating applied onto the tablet core, comprising the application of a further active ingredient layer onto active ingredient-containing pellets having the following structure:
a) an inert core,
b) an active ingredient layer which is applied onto the inert core, and
c) a layer applied onto the active ingredient layer retarding the release of the active ingredient,
wherein the active ingredient is hydromorphone and in which the layer retarding the release of the active ingredient applied onto the active ingredient layer contains as retarding agent ethyl cellulose.

2. Tablet having a tablet core consisting of several active ingredient-containing pellets and one or more pharmaceutically acceptable excipients and at least one coating applied onto the tablet core, wherein the active ingredient-containing pellets contain as active ingredient hydromorphone or a salt or a solvate thereof and have the following structure:
a) an inert core,
b) an active ingredient layer which is applied onto the inert core,
c) a layer applied onto the active ingredient layer retarding the release of the active ingredient, and
d) a further active ingredient layer on the layer retarding the release of the active ingredient,
wherein the layer retarding the release of the active ingredient applied onto the active ingredient layer contains as retarding agent ethyl cellulose.

3. Tablet according to claim 2 containing the active ingredient at a concentration in the range of 2 to 30% by weight, based on the total weight of the tablet.

4. Tablet according to claim 2 or 3, wherein the pharmaceutically acceptable excipients compressed into the tablet core with the pellets are selected from binders, fillers, disintegrants, lubricants, glidants, and mixtures thereof.

5. Tablet according to any one of the preceding claims, wherein a coating is applied onto the tablet core and this coating is not a layer retarding the release of the active ingredient.

6. Active ingredient-containing pellet with the active ingredient hydromorphone, having the following structure:
a) an inert core,
b) an active ingredient layer which is applied onto the inert core, and
c) a layer applied onto the active ingredient layer retarding the release of the active ingredient,
wherein the layer retarding the release of the active ingredient applied onto the active ingredient layer contains as retarding agent ethyl cellulose.

7. Active ingredient-containing pellet according to claim 6, **characterized in that** the inert core has a diameter in the range of 0.2 mm to 1.5 mm.

8. Active ingredient-containing pellet according to claim 6 or 7, **characterized in that** the active ingredient layer releases at least 90 % of the active ingredient within 15 minutes, determined according to the Paddle method of the US Pharmacopeia (100 rpm in 900 ml aqueous buffer, pH value in the range of 1.6 and 7.2 at 37°C).

## Revendications

1. Procédé pour la préparation d'un comprimé comportant un noyau de comprimé constitué de plusieurs granulés contenant une substance active et un ou plusieurs adjuvants pharmaceutiquement acceptables et au moins un enrobage appliqué sur le noyau de comprimé, comprenant l'application d'une autre couche de substance active sur les granulés contenant la substance active et présentant la structure suivante :
a) un noyau inerte,
b) une couche de substance active appliquée sur le noyau inerte,
c) une couche appliquée sur la couche de substance active qui retarde la libération de la substance active,
la substance active étant de l'hydromorphone et la couche de substance active qui retarde la libération de la substance active contient de l'éthylcellulose comme agent retard.

2. Comprimé comportant un noyau de comprimé constitué de plusieurs granulés contenant une substance active et un ou plusieurs adjuvants pharmaceutiquement acceptables et au moins un enrobage appliqué sur le noyau de comprimé, les granulés contenant la substance active présentant comme substance active de l'hydromorphone ou un sel ou un solvate de ceux-ci et présentant la structure suivante :
a) un noyau inerte,
b) une couche de substance active appliquée sur le noyau inerte,
c) une couche appliquée sur la couche de substance active qui retarde la libération de la substance active et la couche appliquée sur la couche de substance active qui retarde la libération de la substance active contenant de l'éthylcellulose comme agent retard.

3. Comprimé selon la revendication 2, qui contient la substance active en une concentration dans une plage de 2 à 30 % en poids par rapport au poids total du comprimé.

4. Comprimé selon la revendication 2 ou 3, dans lequel les adjuvants pharmaceutiquement acceptables qui sont comprimés avec les granulés en noyau de comprimé sont choisis parmi les liants, les agents de charges, les agents de désagrégation, les lubrifiants, les agents glissants et leurs mélanges.

5. Comprimé selon l'une des revendications précédentes, dans lequel un revêtement est appliqué sur le noyau de comprimé et ce revêtement n'est pas une couche retardant la libération de la substance active.

6. Granulé contenant une substance active avec la substance active hydromorphone qui présente la structure suivant :
a) un noyau inerte,
b) une couche de substance active appliquée sur le noyau inerte
c) une couche appliquée sur la couche de substance active qui retarde la libération de la substance active et la couche appliquée sur la couche de substance active qui retarde la libération de la substance active contient de l'éthylcellulose comme agent retard.

7. Granulé contenant une substance active selon la revendication 6, **caractérisé en ce que** le noyau inerte présente un diamètre de l'ordre de 0,2 mm à 1,5 mm.

8. Granulé contenant une substance active selon la revendication 6 ou 7, **caractérisé en ce que** la couche de substance active libère au moins 90 % de la substance active en l'espace de 15 minutes déterminées selon le procédé de Paddle de la pharmacopée US (100 t/m dans 900 ml de tampon aqueux, pH dans une plage de 1,6 à 7,2 à 37° C).
